# EUROPEAN PATENT APPLICATION

(11) **EP 2 548 883 A2**
(43) Date of publication of application: **23.01.2013**
(21) Application number: 11752896.8
(22) Date of filing: 09.03.2011
(51) Int. Cl.: C07K 7/62

(54) **PEPTIDE COMPOUNDS THAT CAN BE USED AS ANTIBACTERIAL AGENTS**

(30) Priority: 10.03.2010 ES 201000349
(71) Applicant: Universidad de Barcelona, 08028 Barcelona (ES)
(72) Inventor: RABANAL ANGLADA, Francesc, E-08859 Begues (ES); CAJAL VISA, Yolanda, E-08859 Begues (ES); GARCIA SUBIRATS, Maria, E-08026 Barcelona (ES); RODRÍGUEZ NÚÑEZ, Montserrat, E-08820 El Prat de Llobregat (ES)
(74) Representative: ABG Patentes, S.L.
(86) International application number: PCT/ES2011/070153
(87) International publication number: WO 2011/110716

(57) **Abstract**

Compounds of formula (I), where: R₀ is (C₈-C₁₁)-branched alkyl, CH₃-(CH₂)ₘ-, CH₃-O-(CH₂CH₂O)₂CH₂-, or phenyl-(CH₂)ₓ-; m = 6-10; x = 1-3; R₁, R₃, R₄, R₇, and R₈ are independently selected from the formula GF-(CH₂)ₙ-; with n = 1-4; and GF = -NH₂ or -NH-C(=NH)-NH₂; R₂ is -CH(CH₃)(OH), -CH(CH₃)₂, -CH₂NH₂ o -CH₂OH; R₅ and R₆ are independently selected among H, -(C₁-C₄)- linear or branched alkyl, -(CH₂)-R₁₀, -CH₂-CH₂-S-CH₃ and -CH-(CH₃)-OH; R₉ is CONH₂, -CH(CH₃)(OH) or CONHR₁₁; R₁₀ is phenyl, 3-indolyl, 4-imidazolyl, 4-hydroyiphenyl, α o β-naphtyl or 2-, 3- or 4-pyridyl; R₁₁ is a specific peptide sequence; u is CH₂ or S; v es NH o S; w is CH₂ or CO; with the condition that when R₉ is CONH₂, then (a) R₅ or R₆ is -CH(CH₃)(OH), or (b) R₅ and R₆ are H; or (c) the configuration of the C bound to R₉ is S or (d) the configuration of the C bound to R₅ is R; and with the condition that when R₉ is -CH(CH₃)OH, then R₈ is GF(CH₂)ₙ where n is 3 and GF is -NH-C(=NH)-NH₂, and R₇ is GF(CH₂)ₙ where n is 2 and GF is NH₂, have been found to be useful in the treatment of bacterial infections.

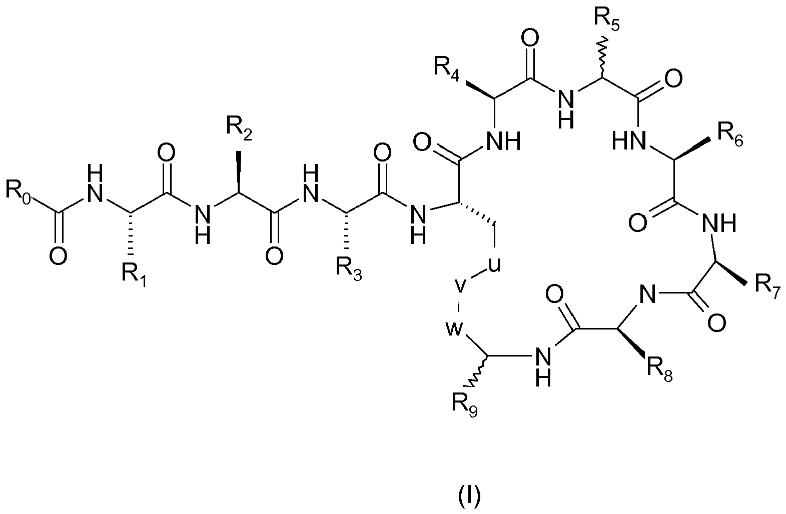

## Description

The present invention relates to new compounds which are active against Gram-positive and some Gram-negative bacteria, to a preparation process thereof, pharmaceutical compositions containing them, and to their use as antibacterials.

### BACKGROUND ART

Disease-causing microorganisms that have become resistant to antibiotic drug therapy are an increasing public health problem. Part of the problem is that certain bacteria and other microorganisms are remarkably capable of developing resistance to antibiotics. Another cause of the problem is the misuse of antibiotics in human and veterinary medicine and in agriculture.

There is substantial concern worldwide with the mounting prevalence of infections caused by multidrug-resistant bacteria, including methicillin-resistant *Staphylococcus aureus,* vancomycin-resistant Enterococci, and certain Gram-negative bacteria such as Pseudomonas aeruginosa, Acinetobacter baumanii and Klebsiella pneumoniae. Such infections are extremely difficult to control, and are prevalent cause of disease and mortality. Conventional antibiotics tipically interact with one or more specific target protein or target receptor, and genetic resistance appears at a rate that depends on many factors, such as the number of proteins or target receptors.

The continuous emergence of bacterial strains that are resistant to conventional antibiotics has led to intensive efforts aimed at the development of novel drugs targeting the bacterial cell membranes, such as antimicrobial peptides (AMP's). AMPs offer a new class of therapeutic agents to which bacteria may not be able to develop genetic resistance, since they mainly act on the lipid component of the cell membranes. Among these compounds, the clinically aproved antibiotic polymyxin B (PxB) is adquiring new therapeutical relevance and is starting to be considered as a representative of a class of antibiotics against multiresistant bacteria.

Polymyxins, and particularly polymyxin B, are a class of antibiotics discovered in 1947 with significant activity against Gram-negative bacteria. Polymyxin B is a lipopeptide antibiotic isolated from *Bacillus polymyxa.* Its basic structure consists of a polycationic peptide ring and a tripeptide side chain with a fatty acid tail. Polymyxin B has re-emerged in medical practice in recent years and its use will likely continue to increase due to the dry antibiotic development pipeline and worldwide increasing prevalence of nosocomial infections caused by multidrug-resistant (MDR) Gram negative bacteria. Polymyxin B and other members of the polymyxin family are drugs of last resort to treat infections caused by multiresistant bacteria and sometimes are the only available active antibiotics. In addition, resistance to polymyxins is rare, and generally adaptive and thus reversible. Polymixin B is also capable of inhibiting the biological activities of bacterial lipopolysaccharide (LPS) through high-affinity binding to the lipid A moiety, being the agent of choice to treat LPS-induced septic shock. Unfortunately, polymyxin B has no activity against Gram-positive and anaerobes. Furthermore, polymyxins have limited use since they show some nefrotoxicity and neurotoxicity.

Thus, there is still a need to find new effective antibacterial agents with activity not only against Gram-negative bacteria but also against Gram-positive bacteria.

### SUMMARY OF THE INVENTION

Inventors have found some peptide compounds with antibiotic activity that act at the level of the lipidic component of the bacterial membrane and which are active against both Gram positive and Gram negative bacteria. These compounds are based on the structure of natural polymyxin. However, unlike polymyxins, these compounds are not only active against Gram-negative bacteria but also against active against Gram positive bacteria in the micromolar range.This is advantageous since they can act in response to infection caused by both type of bacteria.

Thus, an aspect of the present invention is to provide a compound of formula (I), 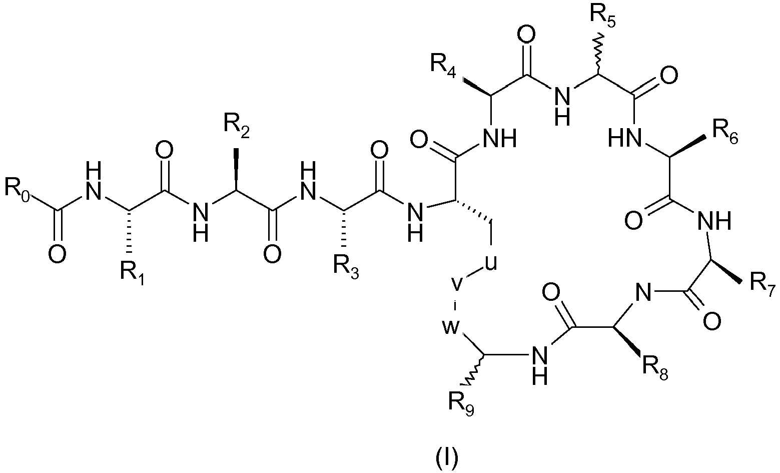 where R₀ is a radical selected from the group consisting of: (C₈-C₁₁)-branched alkyl, CH₃-(CH₂)ₘ-, CH₃-O-(CH₂CH₂O)₂CH₂-, and 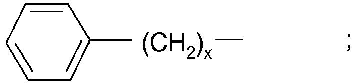 m is an integer from 6 to 10; x is an integer from 1 to 3; R₁, R₃, R₄, R₇, and R₈ are independently selected radicals having the following formula: GF-(CH₂)ₙ-; where n is an integer from 1 to 4; GF is a radical selected from the group consisting of -NH₂ and -NH-C(=NH)-NH₂; R₂ is a radical selected from the group consisting of -CH(CH₃)(OH), -CH(CH₃)₂, -CH₂NH₂ y -CH₂OH; R₅ and R₆ are radicals independently selected from the group consisting of H, linear or branched (C₁-C₄)-alkyl, -(CH₂)-R₁₀, -CH₂-CH₂-S-CH₃ and -CH(CH₃)(OH); R₉ is selected from the group consisting of CONH₂, -CH(CH₃)(OH) and CONHR₁₁; R₁₀ is a radical selected from the group consisting of phenyl, 3-indolyl, 4-imidazolyl, 4-hydroxyphenyl, α or β-naphtyl and 2-, 3- or 4-pyridyl;
R₁₁ is a peptide sequence selected from the group consisting of Ala-Leu-Arg, Ala-Leu-Arg-Ala-Leu-Arg, Gly-Arg-Val-Glu-Val-Leu-Tyr-Arg-Gly-Ser-Trp, Lys-Val-Leu, Lys-Val-Leu-Lys-Val-Leu, Leu-Met-Trp-Trp-Met-Leu, Orn-Orn-Orn, Gln-Arg-Gly-Arg-Ala-Glu-Glu-Val-Tyr-Tyr-Ser-Gly-Thr, and Glu-(γ-spermide)-Arg-Gly-Arg-Ala-Glu-Glu-Val-Tyr-Tyr-Ser-Gly-Thr; u is CH₂ or S; v is NH or S; w is CH₂ or CO; with the proviso that when R₉ is CONH₂, thenone of the following conditions apply: (a) R₅ or R₆ is -CH(CH₃)(OH), (b) R₅ and R₆ are H; (c) the configuration of the C atom bound to R₉ is S (sidechain of D-cysteine) or (d) the configuration of the C atom bound to R₅ is R; and with the proviso that when R₉ is -CH(CH₃)OH (sidechain of threonine), then R₈ is GF(CH₂)ₙ where n is 3 and GF is -NH-C(=NH)-NH₂, and R₇ is GF(CH₂)ₙ where n is 2 and GF is NH₂.

In a preferred embodiment, compounds of formula (I) are those mentioned before where R₂ es -CH(CH₃)(OH).

In an another preferred embodiment, compounds of formula (I) are those where u is CH₂, v is NH, w is CO, R₉ is -CH(CH₃)(OH) and the configuration of the C atom bound to R₉ is S. These compounds have the formula (Ia).

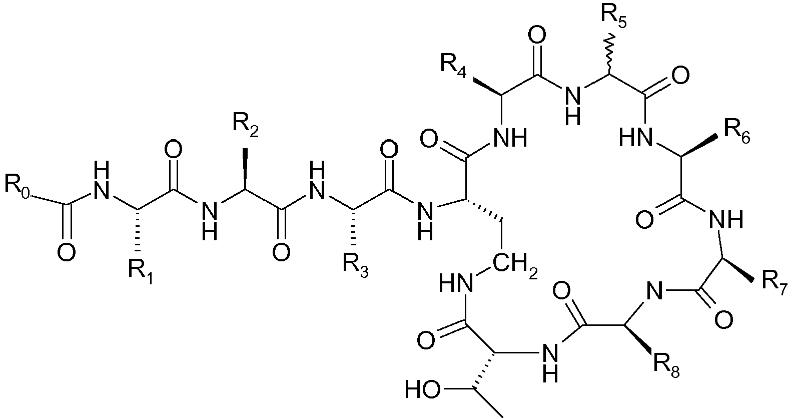

In a more preferred embodiment, the compound of formula (Ia) is nonanoyl-Arg-Thr-Dab-cyclo(4-10)[Dab-Dab-DPhe-Leu-Arg-Dab-Thr], (Ia₁).

The term cyclo(4-10) means that the terminal carboxy group of threonine in position 10 is bound to the side-chain of Dab in position 4 by means of an amide bond, forming a macrocycle.

The configuration of D-amino acids have been indicated with a D. When no indication of the configuration is mentioned , it is understood that the configuration of the amino acid is L.

In an another preferred embodiment, compounds of formula (I) are those where u is S, v is S and w is CH₂, and have the formula (Ib)

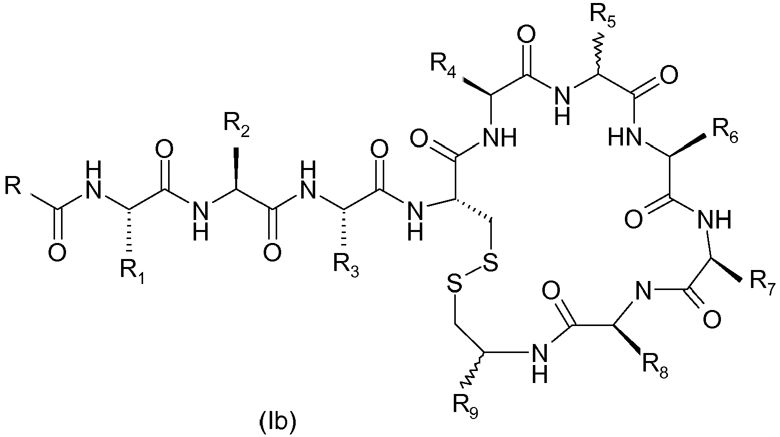

In an another preferred embodiment, compounds of formula (Ib) are those where R₉ es CONHR₁₁ and R₁₁ is a peptide sequence selected from the group that consists of Ala-Leu-Arg, Ala-Leu-Arg-Ala-Leu-Arg, Gly-Arg-Val-Glu-Val-Leu-Tyr-Arg-Gly-Ser-Trp, Lys-Val-Leu, Lys-Val-Leu-Lys-Val-Leu, Leu-Met-Trp-Trp-Met-Leu, Orn-Orn-Orn, Gln-Arg-Gly-Arg-Ala-Glu-Glu-Val-Tyr-Tyr-Ser-Gly-Thr, Glu(γ-spermide)-Arg-Gly-Arg-Ala-Glu-Glu-Val-Tyr-Tyr-Ser-Gly-Thr, y Glu(Arg-Gly-Arg-Ala-Glu-Glu-Val-Tyr-Tyr-Ser-Gly-Thr)-γ-spermide;

In an another even more preferred embodiment, compounds of formula (Ib) are selected from the following list:
nonanoyl-Arg-Thr-Dab-cyclo(S-S)[Cys-Dab-DPhe-Leu-Arg-Dab-Cys]-Ala-Leu-Arg, (Ib₁);
nonanoyl-Arg-Thr-Dab-cyclo(S-S)[Cys-Dab-DPhe-Leu-Arg-Dab-Cys]-Ala-Leu-Arg-Ala-Leu-Arg, (Ib₂);
nonanoyl-Arg-Thr-Dab-cyclo(S-S)[Cys-Dab-DPhe-Leu-Arg-Dab-Cys]-Gly-Arg-Val-Glu-Val-Leu-Tyr-Arg-Gly-Ser-Trp], (Ib₃);
nonanoyl-Arg-Thr-Dab-cyclo(S-S)[Cys-Dab-DPhe-Leu-Arg-Dab-Cys]-Lys-Val-Leu, (Ib₄);
nonanoyl-Arg-Thr-Dab-cyclo(S-S)[Cys-Dab-Phe-Leu-Arg-Dab-Cys]-Lys-Val-Leu-Lys-Val-Leu, (Ib₅);
nonanoyl-Arg-Thr-Dab-cyclo(S-S)[Cys-Dab-DPhe-Leu-Arg-Dab-Cys]-Leu-Met-Trp-Trp-Met-Leu, (Ib₆);
nonanoyl-Arg-Thr-Dab-cyclo(S-S)[Cys-Dab-DPhe-Leu-Arg-Dab-Cys]-Orn-Orn-Orn, (Ib₇);
nonanoyl-Arg-Thr-Dab-cyclo(S-S)[Cys-Dab-DPhe-Leu-Dab-Arg-Cys]-Gln-Arg-Gly-Arg-Ala-Glu-Glu-Val-Tyr-Tyr-Ser-Gly-Thr, (Ib₈); and
nonanoil-Arg-Thr-Dab-ciclo(S-S)[Cys-Dab-DPhe-Leu-Dab-Arg-Cys]-Glu-(γ-spermide)-Arg-Gly-Arg-Ala-Glu-Glu-Val-Tyr-Tyr-Ser-Gly-Thr, (Ib₉).

The term "cyclo(S-S)" implies that a macrocycle is formed by means of disulfide bond between both cysteines.

In an another preferred embodiment, compounds of formula (Ib) are selected from the following list:
nonanoyl-Arg-Thr-Arg-cyclo(S-S)[Cys-Dab-Phe-Leu-Arg-Dab-Cys], (Ib₁₀);
nonanoyl-Arg-Thr-Arg-cyclo(S-S)[Cys-Dab-DPhe-Leu-Arg-Dab-DCys], (Ib₁₁);
nonanoyl-Arg-Thr-Dab-cyclo(S-S)[Cys-Dab-Phe-Leu-Arg-Dab-Cys], (Ib₁₂); and
nonanoyl-Arg-Thr-Dab-cyclo(S-S)[Cys-Dab-DPhe-Leu-Arg-Dab-DCyS], (Ib₁₃).

Particularly good results were achieved with peptides containing a D-cystein at position 10. In an another more preferred embodiment, compounds of formula (Ib) are selected among nonanoyl-Arg-Thr-Arg-cyclo(S-S)[Cys-Dab-DPhe-Leu-Arg-Dab-DCyS], (Ib₁₁); and nonanoyl-Arg-Thr-Dab-cyclo(S-S)[Cys-Dab-DPhe-Leu-Arg-Dab-DCyS], (Ib₁₃). In an another more preferred embodiment, the compound of formula (Ib) is nonanoyl-Arg-Thr-Dab-cyclo(S-S)[Cys-Dab-DPhe-Leu-Arg-Dab-DCyS], (Ib₁₃).

In an another preferred embodiment, compounds of formula (Ib) are those in which R₆ is -(CH)(CH₃)(OH).

In an another preferred embodiment, compounds of formula (Ib) are selected from the following list:
nonanoyl-Arg-Thr-Arg-cyclo(S-S)[Cys-Dab-DPhe-Thr-Arg-Dab-Cys], (Ib₁₄);
nonanoyl-Arg-Thr-Arg-cyclo(S-S)[Cys-Dab-Phe-Thr-Arg-Dab-Cys], (Ib₁₅);
nonanoyl-Arg-Thr-Dab-cyclo(S-S)[Cys-Dab-DPhe-Thr-Arg-Dab-Cys], (Ib₁₆);
nonanoyl-Arg-Thr-Dab-cyclo(S-S)[Cys-Dab-Phe-Thr-Arg-Dab-Cys], (Ib₁₇);
nonanoyl-Arg-Thr-Arg-cyclo(S-S)[Cys-Dab-Trp-Thr-Arg-Dab-Cys], (Ib₁₈);
nonanoyl-Arg-Thr-Dab-cyclo(S-S)[Cys-Dab-Trp-Thr-Arg-Dab-Cys], (Ib₁₉);
nonanoyl-Arg-Thr-Arg-cyclo(S-S)(Cys-Dab-DLeu-Thr-Arg-Dab-Cys], (Ib₂₀);
nonanoyl-Arg-Thr-Dab-cyclo(S-S)[Cys-Dab-DLeu-Thr-Arg-Dab-Cys], (Ib₂₁);
decanoyl-Arg-Thr-Arg-cyclo(S-S)[Cys-Dab-DTrp-Thr-Dab-Dab-Cys], (Ib₂₂);
decanoyl-Arg-Thr-Dab-cyclo(S-S)[Cys-Dab-DLeu-Thr-Arg-Dab-Cys], (Ib₂₃) decanoyl-Arg-Thr-Dab-cyclo(S-S)[Cys-Dab-Trp-Thr-Arg-Dab-Cys], (Ib₂₄) ; and
dodecanoyl-Arg-Thr-Dab-cyclo(S-S)[Cys-Dab-Phe-Thr-Arg-Dab-Cys], (Ib₂₅).

In an another preferred embodiment, compounds of formula (Ib) are those where R₅ or R₆ is hydrogen.

In an another preferred embodiment, compounds of formula (Ib) are selected from the following list:
nonanoyl-Arg-Thr-Arg-cyclo(S-S)[Cys-Dab-Gly-Gly-Arg-Dab-Cys], (Ib₂₆₎;
nonanoyl-Arg-Thr-Arg-cyclo(S-S)[Cys-Dab-Gly-Leu-Arg-Dab-Cys], (Ib₂₇);
nonanoyl-Arg-Thr-Arg-cyclo(S-S)[Cys-Dab-DPhe-Gly-Arg-Dab-Cys], (Ib₂₈) nonanoyl-Arg-Thr-Dab-cyclo(S-S)[Cys-Dab-DPhe-Gly-Arg-Dab-Cys], (Ib₂₉); and
nonanoyl-Arg-Thr-Dab-cyclo(S-S)[Cys-Dab-Gly-Leu-Arg-Dab-Cys], (Ib₃₀).

In an another preferred embodiment, compounds of formula (Ib) are selected from the following list:
octanoyl-Arg-Thr-Arg-cyclo(S-S)[Cys-Dab-DPhe-Leu-Arg-Dab-Cys], (Ib₃₁);
(S)-6-methyl heptanoyl-Arg-Thr-Arg-cyclo(S-S)[Cys-Dab-DPhe-Leu-Arg-Dab-Cys], (Ib₃₂);
(S)-6-methyl octanoyl-Arg-Thr-Arg-cyclo(S-S)[Cys-Dab-DPhe-Leu-Arg-Dab-Cys], (Ib₃₃);
octanoyl-Arg-Thr-Dab-cyclo(S-S)[Cys-Dab-DPhe-Leu-Arg-Dab-Cys], (Ib₃₄);
(S)-6-methyl heptanoyl-Arg-Thr-Dab-cyclo(S-S)[Cys-Dab-DPhe-Leu-Arg-Dab-Cys], (Ib₃₅); and
(S)-6-methyl octanoyl-Arg-Thr-Dab-cyclo(S-S)[Cys-Dab-DPhe-Leu-Arg-Dab-Cys], (Ib₃₆).

The compounds of formula (I) more preferred are the ones of the following list:
nonanoyl-Arg-Thr-Dab-cyclo(4-10)[Dab-Dab-DPhe-Leu-Arg-Dab-Thr], (Ia₁);
nonanoyl-Arg-Thr-Dab-cyclo(S-S)[Cys-Dab-DPhe-Leu-Arg-Dab-Cys]-Gly-Arg-Val-Glu-Val-Leu-Tyr-Arg-Gly-Ser-Trp], (Ib₈);
nonanoyl-Arg-Thr-Dab-cyclo(S-S)[Cys-Dab-DPhe-Leu-Arg-Dab-Cys]-Leu-Met-Trp-Trp-Met-Leu, (Ib₆); and
nonanoyl-Arg-Thr-Dab-cyclo(S-S)[Cys-Dab-DPhe-Leu-Arg-Dab-DCys], (Ib₁₃).

Compounds of the present invention can be prepared by solid phase Fmoc/tBu synthesis. The synthesis protocol for each amino acid consisted of the following steps: (i) resin washing with N,N-dimethylformamide (DMF) several times; ii) treatment with 20 % piperidine/DMF; iii) washing with DMF several times; iv) acylation with Fmoc-protected amino acid and 2-(1 H-benzotriazol-1-yl)-1,1,3,3-tetramethyluronium hexafluorophosphate /diisopropylethylamine (HBTU/DIEA) in DMF; v) washing with DMF several times and dichloromethane (CH₂Cl₂) several times; and vi) DMF washing several times.

The peptides obtained by the previous processes can be purified by preparative HPLC. A final purity greater than 90% can be achieved by this method, but preferably higher than 95%.

Compounds of the present invention are readily prepared by chemical synthesis according to the described method whereas commercial polymyxin is obtained by a fermentation process.

Another aspect of the present invention are the compounds of formula (I), for use as antibacterial agents against Gram positive bacteria. Among Gram positive bacteria that are relevant for medical purposes, several genus such as Bacillus, Listeria, Staphylococcus, Micrococcus, Streptococcus, Enterococcus, Clostridium, Mycoplasma and Actinobacteria are well known.

In a preferred embodiment, Gram positive bacteria are selected among *Micobacterium phlei, Staphylococcus aureus,* and *Micrococcus luteus.* In a particular embodiment, Gram positive bacteria are selected among Micobacterium phlei ATCC41423, Staphylococcus aureus ATCC 6538 and Micrococcus luteus ATCC 9341.

This aspect of the invention can also be formulated as the use of a compound such as those defined above for the preparation of a medicament for the treatment of a bacterial infection caused by Gram positive bacteria in a mammal including a human.

The invention also relates to a method of treatment and/or prophylaxis of a mammal, including a human, suffering from or being susceptible to bacterial infection caused by Gram positive bacteria, in particular to one of the infections mentioned above, said method comprising the administration to said patient of a therapeutically effective amount of the compounds of the present invention, together with pharmaceutically acceptable excipients or carriers.

It is also part of the invention the compounds of formula (I) as defined above for use as antibacterial agents against Gram negative bacteria. Medically relevant Gram negative bacteria include Escherichia coli, Salmonella, Enterobacteriaceae, Pseudomonas, Moraxella, Helicobacter, Legionella, Hemophilus influenzae, Klebsiella pneumoniae, Proteus mirabilis, Enterobacter cloacae, Serratia marcescens, and Acinetobacter baumannii.

In a preferred embodiment, Gram negative bacteria are selected among Salmonella typhimurium, Pseudomonas aeruginosa, Escherichia coli, and Acinetobacter sp. In a particular embodiment, the Gram negative bacteria are selected among Salmonella typhimurium 14028, Pseudomonas aeruginosa 9027, Escherichia coli 8739 and Acinetobacter sp ATCC 5798. This aspect of the invention can also be formulated as use of a compound of formula (I) as defined above for the preparation of a medicament for the treatment of a bacterial infection caused by a Gram negative bacteria in a mammal, including a human.

The invention also relates to a method of treatment and/or prophylaxis of a mammal, including a human, suffering from or being susceptible to bacterial infection caused by Gram negative bacteria, in particular to one of the infections mentioned above, said method comprising the administration to said patient of a therapeutically effective amount of the compounds of formula (I) as defined above, together with pharmaceutically acceptable excipients or carriers. Compounds of the present invention can be used in the same manner as other known antibacterial agents. They may be used alone or in combination with other suitable bioactive compounds. In a particular embodiment, compounds of formula (I) are used in the treatment of bacteremia and/or septicemia following infection by Gram negative bacteria or Gram positive bacteria, administered alone or in combination with conventional antibiotics. In a preferred embodiment, the compounds of formula (I) of the present invention are used topically or orally for the descontamination of the digestive tract before surgery.

As mentioned above these compounds are advantageous since they are active against a broad bacteria spectrum, and thus they presumably act at the membrane level, they can be more effective than other antibiotics that act on a specific receptor or an enzyme in front of the resistance to antibiotics.

A further aspect of the present invention relates to a pharmaceutical composition comprising a therapeutically effective amount of the compounds of formula (I), together with appropriate amounts of pharmaceutical excipients or carriers.

The pharmaceutical compositions may be prepared by combining the compounds of formula (I) of this invention with solid or liquid pharmaceutically acceptable excipients or carriers, following, in accordance with standard pharmaceutical practice.

The pharmaceutical compositions of this invention may be administered in standard manner for the disease condition that it is desired to treat, for example by oral, parenteral, inhalatory, rectal, transdermal or topical administration. In therapeutic use for treating, or combating bacterial infections in patients such as humans and other animals that can be diagnosed with bacterial infections, the compounds or pharmaceutical compositions thereof, preferably, will be administered at a dosage to obtain and maintain a concentration, i.e. an amount or blood-level of active component in the patient undergoing treatment which will be antibacterially effective. Generally such antibacterially effective amount of dosage of active component will be in the range of about 0.1 to about 100 mg/Kg, more preferably about 3.0 to about 50 mg/Kg of body weight/day. It is to be understood that the dosages may vary depending upon the requirements of the patient, the severity of the bacterial infection being treated, and the particular compound being used.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. In addition, the present invention covers all posible combinations of particular and preferred embodiments herein indicated.

### EXAMPLES

Abbreviations: Boc, tert-butoxycarbonyl; Dab: 2,4-diaminobutyric acid; DIEA, N,N-diisopropylethylamine; Dde, N -[1-(4,4-dimethyl-2,6-dioxocyclohex-1-ylidene)ethyl]; DIPCDI, N,N'-diisopropylcarbodiimide; DMF, N,N-dimethylformamide; ES, electrospray; Fmoc, 9-fluorenylmethoxycarbonyl; HATU, 2-(7-aza-1H-benzotriazole-1-yl)-1,1,3,3-tetramethyluronium hexafluorophosphate; HBTU, 2-(1H-benzotriazol-1-yl)-1,1,3,3-tetramethyluronium hexafluorophosphate; HOBt, 1-hydroxybenzotriazole; HPLC,high performance liquid chromatography; MALDI-TOF, matrix-assisted laser desorption ionization time-of-flight; MS, mass spectrometry; MIC, minimum inhibitory concentration; MW, molecular weight; Pbf, 2,2,4,6,7-pentamethyldihydrobenzofuran-5-sulfonyl; TFA, trifluoroacetic acid; Trt, trityl

### General methods

To prepare the compounds of the Examples, general protocols of Solid Phase Fmoc/tBu synthesis were used.

The Fmoc/tBu synthesis protocol for each synthetic cycle consisted of the following steps: (i) resin washing with DMF (5 x 30 s); (ii) treatment with 20 % piperidine/DMF (1 x 1 min + 2 × 10 min, Fmoc removal); (iii) washing with DMF (5 x 30 s); (iv) acylation with Fmoc-protected amino acid (3 times of excess) with activating reagent HBTU/DIEA (3 and 6 times of excess, respectively) in the minimum amount of DMF; (v) washing with DMF (5 x 30 s) and CH₂Cl₂ (5 x 30 s); (vi) Kaiser's test (with a peptide-resin sample); (vii) DMF washing (5 x 30 s).

General cleavage and full deprotection of the peptides was carried out by treatment with TFA/thioanisole/1,2-ethanedithiol/triisopropylsilane/water (70:10:10:1:3.5; 3h) in a reaction vessel. The TFA solution was filtered off. The resins were rinsed twice with an additional 0.5 mL of TFA mixture, and the rinses were combined. The resulting cleavage solution was added to cold ether (TFA mixture:ether in 1:20 ratio) to precipitate the peptides. Peptide crudes were washed additionally with ether (3 times), ether was decanted and the solid dried out in air.

Peptide crudes were then dissolved in a 10% DMSO solution in water to a concentration of 1 mM or slightly lower for intramolecular disulfide bond formation. The solution was stirred in an open system for 12-36 h. Cyclization reaction took place by air oxidation and was monitored by HPLC and MS.

Purification was carried out by preparative HPLC. In the Examples, a Varian Pro-star 200 prep-system with self-packed Varian Load & lock C18 column (250 x 2.5 mm,10 µm) eluted with H₂O-acetonitrile-0.1% TFA gradient mixtures and UV detection at 220 nm was used.

Peptides were characterized by MALDI-TOF mass spectrometry with a PerSeptive Biosystems, Voyager-DE.

Homogeneity of purified peptides was assessed by analytical HPLC employing Merck or Kromasil C18 reverse phase columns (4 x 250 mm, 5 µm of particle diameter and a pore size of 120 A). Elution was carried out at 1 ml·min⁻¹ flow with mixtures of H₂O-0.1% TFA and MeCN-0.1% TFA and UV detection at 220 nm.

Example 1: Preparation of nonanoyl-Arg-Thr-Dab-cyclo(S-S)[Cys-Dab-DPhe-Leu-Arg-Dab-Cys]-Gly-Arg-Val-Glu-Val-Leu-Tyr-Arg-Gly-Ser-Trp] (Ib₃), (compound of formula (Ib) with R₀ = CH₃(CH₂)₇-, R₁ =-CH₂CH₂CH₂NHC(=NH)-NH₂ (side chain of Arg), R₂ = -CH(CH₃)OH, (side chain of Thr), R₃ =CH₂CH₂NH₂ (side chain of Dab), R₄ = -CH₂CH₂NH₂ (side chain of Dab), R₅ =-CH₂Ph (side chain of DPhe), R₆ = -CH₂CH(CH₃)₂ (side chain of Leu), R₇ = - CH₂CH₂CH₂NHC(=NH)-NH₂ (side chain of Arg), and R₈ = -CH₂CH₂NH₂ (sidechain of Dab), R₉ = Gly-Arg-Val-Glu-Val-Leu-Tyr-Arg-Gly-Ser-Trp

Protected amino acids: Fmoc-Cys(Trt)-OH, Fmoc-Dab(Boc)-OH, Fmoc-Arg(Pbf)-OH, Fmoc-Leu-OH, Fmoc-DPhe-OH, Fmoc-Thr(tBu)-OH, Fmoc-Gly-OH, Fmoc-Val-OH, Fmoc-Glu(tBu)-OH, Fmoc-Tyr(tBu)-OH, Fmoc-Ser(tBu)-OH, Fmoc-Trp(Boc)-OH.

A manual peptide synthesis was performed following standard Fmoc/tBu procedures in polypropylene syringes fitted with a polyethylene disc. The Fmoc-Rink resin (600mg, 0.282 mmole, f= 0.47mmole/g of resin) was used. The amino acids of the sequence were added following a Fmoc/tBu solid phase synthesis protocol as described above. Once the full sequence was assembled, nonanoic acid (134 µl, 0.846mmole, 3 times of excess) was coupled with HBTU/DIEA (3 and 6 times of excess, respectively) in the minimum amount of DMF. After completion of the reaction, the resin was washed with DMF (5 x 30 s) and CH₂Cl₂ (5 × 30s).
The weight of crude peptide after cleavage and disulfide bond formation was 300mg (yield 90%). Purification by HPLC yielded 30 mg of pure peptide (yield 10%).

Characterization of the purified peptide: Homogeneity (by area integration of HPLC trace) >95%; ESI: m/z 528.95 ([M+5H⁺]^{5₊}), 660.90 ([M+4H⁺]^{4₊}), 440.95 ([M+6H⁺]^{6₊},) , 881.00([M+3H⁺]^{3₊}). MW: found 2639.75 (expected 2639.39).

Example 2: Preparation of nonanoyl-Arg-Thr-Dab-cyclo(S-S)[Cys-Dab-DPhe-Leu-Ara-Dab-Cysl-Leu-Met-Trp-Trp-Met-Leu (Ib₆), compound of formula (Ib) with R₀ = CH₃(CH₂)₇-, R₁ =-CH₂CH₂CH₂NHC(=NH)-NH₂ (side chain of Arg), R₂ = -CH(CH₃)OH, (side chain of Thr), R₃ =-CH₂CH₂NH₂ (side chain of Dab), R₄ = - CH₂CH₂NH₂ (side chain of Dab), R₅ =-CH₂Ph (side chain of DPhe), R₆ = - CH₂CH(CH₃)₂ (side chain of Leu), R₇ = -CH₂CH₂CH₂NHC(=NH)-NH₂ (side chain of Arg), and R₈ = -CH₂CH₂NH₂ (side chain of Dab), R₉ = Leu-Met-Trp-Trp-Met-Leu

Protected amino acids: Fmoc-Cys(Trt)-OH, Fmoc-Dab(Boc)-OH, Fmoc-Arg(Pbf)-OH, Fmoc-Leu-OH, Fmoc-DPhe-OH, Fmoc-Thr(tBu)-OH, Fmoc-Met-OH, Fmoc-Trp(Boc)-OH.

A manual peptide synthesis was performed following standard Fmoc/tBu procedures on Fmoc-Rink resin as described above in Example 1.

Characterization of purified peptide: Homogeneity (by area integration of HPLC trace) >95%; ESI: m/z 550.15 ([M+4H⁺]⁴⁺), 440.15 ([M+5H⁺]⁵⁺), 733.35.00([M+3H⁺]³⁺). MW: found 2196.6 (expected 2197.04).

Example 3: Preparation of nonanoyl-Arg-Thr-Dab-cyclo(S-S)Cys-Dab-DPhe-Leu-Arg-Dab-DCys], ((Ib13), compound of formula (Ib) with R₀ = CH₃(CH₂)₇-, R₁ =-CH₂CH₂CH₂NHC(=NH)-NH₂ (side chain of Arg), R₂= -CH(CH₃)OH, (side chain of Thr), R₃ =CH₂CH₂NH₂ (side chain of Dab), R₄ = -CH₂CH₂NH₂ (side chain of Dab), R₅ =-CH₂Ph (side chain of DPhe), R₆ = -CH₂CH(CH₃)₂ (side chain of Leu), R₇ = -CH₂CH₂CH₂NHC(=NH)-NH₂ (side chain of Arg), and R₈= - CH₂CH₂NH₂ (side chain of Dab), R₉ = CONHR₁₁, and R₁₁ = H

Protected amino acids: Fmoc-Cys(Trt)-OH, Fmoc-Dab(Boc)-OH, Fmoc-Arg(Pbf)-OH, Fmoc-Leu-OH, Fmoc-DPhe-OH, Fmoc-Thr(tBu)-OH, Fmoc-DCys(Trt)-OH

A manual peptide synthesis was performed following standard Fmoc/tBu procedures on Fmoc-Rink resin as described above in Example 1.

Characterization of purified peptide: Homogeneity (by area integration of HPLC trace) >95%; MALDI-TOF: m/z 1336.68 ([M+H]⁺,100%), 1359.49 ([M+Na]⁺, 17%).

Example 4: Preparation of nonanoyl-Arg-Thr-Dab-cyclo(S-S)[Cys-Dab-DPhe-Thr-Arg-Dab-Cys] ((Ib₁₆), compound of formula (Ib) with R₀ = CH₃(CH₂)₇-, R₁ =-CH₂CH₂CH₂NHC(=NH)-NH₂ (side chain of Arg), R₂= -CH(CH₃)OH, (side chain of Thr), R₃ =-CH₂CH₂NH₂ (side chain of Dab), R₄ = -CH₂CH₂NH₂ (side chain of Dab), R₅ = -CH₂Ph (side chain of DPhe), R₆ =-CH(CH₃)(OH), (side chain of Thr), R₇ = -CH₂CH₂CH₂NHC(=NH)-NH₂ (side chain of Arg), and R₈ = -CH₂CH₂NH₂ (sidechain of Dab), R₉ = CONHR₁₁, R₁₁ = H

Protected amino acids: Fmoc-Cys(Trt)-OH, Fmoc-Dab(Boc)-OH, Fmoc-Arg(Pbf)-OH, Fmoc-DPhe-OH, Fmoc-Thr(tBu)-OH,

A manual peptide synthesis was performed following standard Fmoc/tBu procedures on Fmoc-Rink resin as described above in Example 1.

Characterization of purified peptide: Homogeneity (by area integration of HPLC trace) >95%; MALDI-TOF: m/z 1324.24 ([M+H]⁺,100%), 1347.45 ([M+Na]⁺, 16%).

Example 5: Preparation of nonanoyl-Arg-Thr-Dab-cyclo(*S-S*)[Cys-Dab-DPhe-Gly-Arg-Dab-Cys] ((Ib₂₉), compound of formula (Ib) with R₀ = CH₃(CH₂)₇-, R₁ =-CH₂CH₂CH₂NHC(=NH)-NH₂ (side chain of Arg), R₂ = -CH(CH₃)OH, (side chain of Thr), R₃ =-CH₂CH₂NH₂ (side chain of Dab), R₄ = -CH₂CH₂NH₂ (side chain of Dab), R₅ =-CH₂Ph (side chain of DPhe), R₆ =-H, (side chain of Gly), R₇=-CH₂CH₂CH₂NHC(=NH)-NH₂ (side chain of Arg), and R₈ = -CH₂CH₂NH₂ (side chain of Dab), R₉ = CONHR₁₁, y R₁₁ = H

Protected amino acids: Fmoc-Cys(Trt)-OH, Fmoc-Dab(Boc)-OH, Fmoc-Arg(Pbf)-OH, Fmoc-DPhe-OH, Fmoc-Thr(tBu)-OH, Fmoc-Gly-OH.

A manual peptide synthesis was performed following standard Fmoc/tBu procedures on Fmoc-Rink resin as described above in Example 1.

Characterization of purified peptide: Homogeneity (by area integration of HPLC trace) >95%; MALDI-TOF: m/z 1280.59 ([M+H]⁺,100%), 1284.45 ([M+Na]⁺, 18%).

Example 6: Preparation of nonanoyl-Arg-Thr-Dab-cyclo(4-10)[Dab-Dab-DPhe-Leu-Arg-Dab-Thr] ((Ia₁), compound of formula (Ia) with R₀ = CH₃(CH₂)₇-, R₁ =-CH₂CH₂CH₂NHC(=NH)-NH₂ (side chain of Arg), R₂= -CH(CH₃)OH, (side chain of Thr), R₃ =-CH₂CH₂NH₂ (side chain of Dab), R₄ = -CH₂CH₂NH₂ (side chain of Dab), R₅ =-CH₂Ph (side chain of DPhe), R₆ = -CH₂CH(CH₃)₂ (side chain of Leu), R₇= -CH₂CH₂CH₂NHC(=NH)-NH₂ (side chain of Arg), and R₈ = -CH₂CH₂NH₂ (side chain of Dab),

Protected amino acids: Fmoc-Arg(Pbf)-OH, Fmoc-Dab(Boc)-OH, Fmoc-Dab(Dde)-OH, Fmoc-DPhe-OH, Fmoc-Leu-OH Fmoc-Thr(tBu)-OH,.

A manual peptide synthesis was performed following standard Fmoc/tBu procedures in polypropylene syringes fitted with a polyethylene disc. The Clorotrytil chloride resin (800mg,0.48 mmole, f= 0.6mmole/g of resin) was used. The amino acids of the sequence were added following a Fmoc/tBu solid phase synthesis protocol as described above. The bridging Dab⁴ was introduced as Fmoc-Dab(Dde)-OH . Once the full sequence was assembled, nonanoic acid (228 µl, 1.44 mmole, 3 times of excess) was coupled with HBTU/DIEA (3 and 6 times of excess, respectively) in the minimum amount of DMF. After completion of the reaction, the resin was washed with DMF (5 x 30 s) and CH₂Cl₂ (5 x 30 s).

The protected peptide resin was treated with hydrazine (1% in DMF) to remove the Dde protecting group. Peptide was then detached from the resin with a mild TFA treatment (1% in CH₂Cl₂) thus keeping the rest of side chain protecting groups (tBu-type and Pbf groups). The cyclization between Dab⁴ and the C-terminal Thr¹⁰ was carried out in DMF:CHCl₃=1:1 solution (peptide concentration 0.3M) with HATU:HOBt:DIEA (1.1:1.1:2.2 equiv.) at room temperature for 48h. The crude, protected cyclic peptide was purified on a silica column before the final TFA cleavage to remove the rest of the side-chain protective groups.

The weight of crude peptide was 130 mg (yield 21 %). Purification by HPLC yielded 11 mg (yield 8.5%).

Characterization of purified peptide: Homogeneity (by area integration of HPLC trace) >91'8%; MALDI-TOF: m/z 1316.89 ([M+H]⁺, 100%), 1297.98 ([M+H-H₂O]⁺, 68%) 1339.65 ([M+Na]⁺, 19%).

Example 7: Preparation of nonanoyl-Arg-Thr-Dab-cyclo(S-S)[Cys-Dab-Phe-Leu-Arg-Dab-Cys] (Ib₁₂); compound of formula (Ib) with Ro= CH₃(CH₂)₇-, R₁ = - CH₂CH₂CH₂NHC(=NH)-NH₂ (side chain of Arg), R₂= -CH(CH₃)OH, (side chain of Thr), R₃ =- CH₂CH₂NH₂ (side chain of Dab), R₄ = -CH₂CH₂NH₂ (side chain of Dab), R₅ = -CH₂Ph (side chain of Phe), R₆ = -CH₂CH(CH₃)₂ (side chain of Leu), R₇ = -CH2CH2CH2NHC(=NH)-NH2 (side chain of Arg), R₈ = -CH₂CH₂NH₂ (side chain of Dab), R₉ = CONHR₁₁, yR₁₁ = H

Protected amino acids: Fmoc-Cys(Trt)-OH, Fmoc-Dab(Boc)-OH, Fmoc-Arg(Pbf)-OH, Fmoc-Leu-OH, Fmoc-Phe-OH, Fmoc-Thr(tBu)-OH, Fmoc-Cys(Trt)-OH

A manual peptide synthesis was performed following standard Fmoc/^{t}Bu procedures on Fmoc-Rink resin as described above in Example 1.

Characterization of purified peptide: Homogeneity (by area integration of HPLC trace) >95%; MALDI-TOF: m/z 1336.30 ([M+H]⁺,100%), 1358.40 ([M+Na]⁺, 15%).

Example 8: Preparation of nonanoyl-Arg-Thr-Dab-cyclo(S-S)[Cys-Dab-Gly-Leu-Arg-Dab-Cys], (Ib₃₀), compound of formula (Ib) with Ro = CH₃(CH₂)₇-, R₁ = - CH₂CH₂CH₂NHC(=NH)-NH₂ (side chain of Arg), R₂= -CH(CH₃)OH, (side chain of Thr), R₃ =- CH₂CH₂NH₂ (side chain of Dab), R₄ = -CH₂CH₂NH₂ (side chain of Dab), R₅ = H (side chain of Gly), R₆ = -CH₂CH(CH₃)₂ (side chain of Leu), R₇ = - CH₂CH₂CH₂NHC(=NH)-NH₂ (side chain of Arg), R₈ = -CH₂CH₂NH₂ (side chain of Dab), R₉ = CONHR₁₁, yR₁₁ = H

Protected amino acids: Fmoc-Cys(Trt)-OH, Fmoc-Dab(Boc)-OH, Fmoc-Arg(Pbf)-OH, Fmoc-Gly-OH, Fmoc-Leu-OH, Fmoc-Thr(tBu)-OH, Fmoc-Cys(Trt)-OH

A manual peptide synthesis was performed following standard Fmoc/^{t}Bu procedures on Fmoc-Rink resin as described above in Example 1.

Characterization of purified peptide: Homogeneity (by area integration of HPLC trace) >95%; MALDI-TOF: m/z 1246'03 ([M+H]⁺,100%).

Example 9: Preparation of nonanoyl-Arg-Thr-Dab-cyclo(S-S)[Cys-Dab-DLeu-Thr-Arg-Dab-Cysl, (Ib₂₁), compound of formula (Ib) with Ro = CH₃(CH₂)₇-, R₁ = - CH₂CH₂CH₂NHC(=NH)-NH₂ (side chain of Arg), R₂= -CH(CH₃)OH, (side chain of Thr), R₃ =- CH₂CH₂NH₂ (side chain of Dab), R₄ = -CH₂CH₂NH₂ (side chain of Dab), R₅ = CH₂CH(CH₃)₂ (side chain of _{D}Leu), R₆ =-CH(CH₃)OH, (side chain of Thr), R₇ = -CH₂CH₂CH₂NHC(=NH)-NH₂ (side chain of Arg), R₈ = -CH₂CH₂NH₂ (side chain of Dab), R₉ = CONHR₁₁, y R₁₁ = H

Protected amino acids: Fmoc-Cys(Trt)-OH, Fmoc-Dab(Boc)-OH, Fmoc-Arg(Pbf)-OH, Fmoc-DLeu-OH, Fmoc-Thr(tBu)-OH, Fmoc-Cys(Trt)-OH

A manual peptide synthesis was performed following standard Fmoc/^{t}Bu procedures on Fmoc-Rink resin as described above in Example 1.

Characterization of purified peptide: Homogeneity (by area integration of HPLC trace) >95%; MALDI-TOF: m/z 1291.70 ([M+H]⁺,100%), 1312.76 ([M+Na]⁺, 16%).

### Example 10: Antibacterial test

The antibacterial activity of the lipopeptides was measured in sterile 96-well plates (Corning Costar 3598 microtiter plates). Samples of a final volume of 200µL were prepared as follows: aliquots (100 *µ*L) of a suspension containing bacteria at a concentration of 10⁵ colony-forming units/mL in culture medium (MH, Muller Hinton Broth, Difco, USA), adjusted at pH 7.4, were added to 100µL of solution containing the peptide prepared from a stock solution of 1 mg/mL peptide in water in serial 2-fold dilutions in MH broth adjusted to pH 7.4 (Jorgensen and Turnide, 2003).

Inhibition of bacterial growth was determined by measuring the absorbance at 492 nm with a Absorbance Microplate reader ELx 800 (Bio-tek Instruments) after an incubation of 24-48 h at 37 °C. Antibacterial activities are expressed as the MIC, the concentration at which no growth is observed after 24-48 h of incubation.

Microorganisms were cultured on Tryptycase Soy Broth (Pronadisa, Barcelona) and incubated at 37 °C until bacterial growth was observed. Then a loopful was streaked on Trypticase Soy Agar (Pronadisa, Barcelona) and incubated at 37°C until colony formation occurs. Microorganisms were preserved on cryobilles (EAS laboratoire, France) at -20°C.

Bacterial strains used for the antibacterial activity tests were obtained from the American Type Culture Collection (ATCC, Rockville, MD, USA):
Escherichia coli ATCC 8739
Pseudomonas aeruginosa ATCC 9027
Salmonella typhimurium ATCC 14028
Acinetobacter sp ATCC 5798
Staphylococcus aureus ATCC 6538
Mycobacterium phlei ATCC 41423
Microccous luteus ATCC 9341

The results are shown in Table 1 and Table 2.

**Table 1: Gram positive antibacterial activity (MIC, in µg/ml)**

| Compound (µg/ml) | Micobacterium phlei | Micrococcus luteus | Staphylococcus aureus |
|---|---|---|---|
| (Ia₁) | 8-16 | 16 | 32 |
| (Ib₃) | 16 | 8-16 | 16 |
| (Ib₆) | >32 | 8 | 16 |
| (Ib₁₃) | 8-16 | 8 | 16 |
| PxB P1004 (for comparison) | 32 | 32 | >32 |

**Table 2: Gram negative antibacterial activity (MIC, in µg/ml)**

| Compuesto (µg/ml) | Salmonella typhimurium 14028 | Pseudomonas aeruginosa 9027 | Escherichia coli 8739 | Acinetobacter sp ATCC 5798 |
|---|---|---|---|---|
| (Ia₁) | 8-16 | 4 | 4 | 8 |
| (Ib₃) | 16 | >32 | 16 | 4 |
| (Ib₆) | >32 | >32 | >32 | 8-16 |
| (Ib₁₃) | 16 | 4 | 8 | 16 |
| PxB (comparativo) | 1 | 2 | 1-2 | 0.5 |

Control (polymyxin B): (S)-6-methyloctanoyl-Dab-Thr-Dab-cyclo(4-10)[Dab-Dab-DPhe-Leu-Dab-Dab-Thr].

The molecular weight of the compounds of this invention is higher that that of PxB. This fact means that the activity expressed in micromolar units instead of µg/ml is also higher.

Hence, the above activity results are displayed below in micromolar units for a better comparison of the results.

**Table 3: Gram positive antibacterial activity (MIC, in micromolar units)**

| Compound (microM) | Micobacterium phlei | Micrococcus luteus | Staphylococcus aureus |
|---|---|---|---|
| (Ia₁) | 6.08-12.16 | 12.16 | 24.32 |
| (Ib₃) | 6.06 | 3.03-6.06 | 6.06 |
| (Ib₆) | >14.56 | 3.64 | 7.28 |
| (Ib₁₃) | 5.99-11.98 | 5.99 | 11.98 |
| PxB P1004 (for comparison) | 26.90 | 26.90 | >26.90 |

**Table 4: : Gram negative antibacterial activity (MIC, in micromolar)**

| Compound (microM) | Salmonella typhimurium 14028 | Pseudomonas aeruginosa 9027 | Escherichia coli 8739 | Acinetobacter sp ATCC 5798 |
|---|---|---|---|---|
| (Ia₁) | 6.08-12.16 | 3.04 | 3.04 | 6.08 |
| (Ib₃) | 6.06 | >12.12 | 6.06 | 1.51 |
| (Ib₆) | >14.56 | >14.56 | >14.56 | 3.64-7.28 |
| (Ib₁₃) | 11.98 | 2.99 | 5.99 | 11.98 |
| PxB (for comparison) | 0.84 | 1.68 | 0.84-1.68 | 0.42 |

In addition, it is worth mentioning that the best results have been found with models that have a therapeutic activity. Thus, Acinetobacter sp ATCC 5798 is a model for Acinetobacter baumannii which is one of the most problematic bacteria in nosocomial infections. Staphylococcus aureus is one of the most important bacteria in relation to resistance to antibiotics and Micobacterium phlei is a non-patogenic model of the causal agent of tuberculosis, Micobacterium tuberculosis.

These results show that the new compounds (I) show antibacterial activity at a micromolar level both in Gram positive bacteria and in Gram negative bacteria (in the latter case, slightly higher that the MIC of natural polymyxin, but natural polymyxin does not show activity against Gram positive bacteria). Therefore, the new compounds have a greater activity spectrum since the market available antibiotics (natural polymyxin and daptomicin) are only active against a type of bacteria, Gram negative or Gram positive, respectively.

## Claims

1. A compound of formula (I), 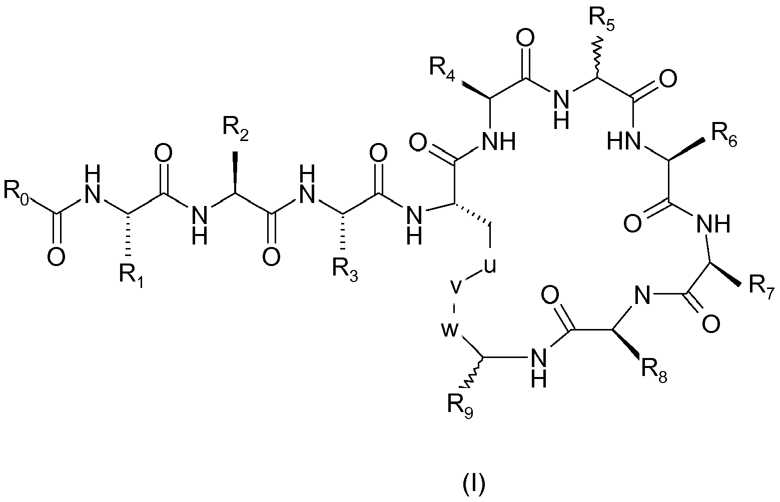 where:
R₀ is a radical selected from the group consisting of: (C₈-C₁₁)-branched alkyl, CH₃-(CH₂)ₘ-, CH₃-O-(CH₂CH₂O)₂CH₂-, and 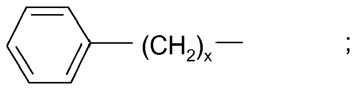
m is an integer from 6 to 10;
x is an integer from 1 to 3;
R₁, R₃, R₄, R₇, and R₈ are independently selected radicals having the following formula:
GF-(CH₂)n-;
where n is an integer from 1 to 4; and GF is a radical selected from the group consisting of -NH₂ and -NH-C(=NH)-NH₂;
R₂ is a radical selected from the group consisting of -CH(CH₃)(OH), -CH(CH₃)₂, -CH₂NH₂ and -CH₂OH;
R₅ and R₆ are radicals independently selected from the group consisting of H, - (C₁-C₄)- linear or branched alkyl, -(CH₂)-R₁₀, -CH₂-CH₂-S-CH₃ and -CH(CH₃)(OH);
R₉ is selected from the group consisting of CONH₂, -CH(CH₃)(OH) and CONHR₁₁;
R₁₀ is a radical selected from the group consisting of phenyl, 3-indolyl, 4-imidazolyl, 4-hydroxyphenyl, α or β-naphtyl and 2-, 3- or 4-pyridyl;
R₁₁ is a peptide sequence selected from the group consisting of Ala-Leu-Arg, Ala-Leu-Arg-Ala-Leu-Arg, Gly-Arg-Val-Glu-Val-Leu-Tyr-Arg-Gly-Ser-Trp, Lys-Val-Leu, Lys-Val-Leu-Lys-Val-Leu, Leu-Met-Trp-Trp-Met-Leu, Orn-Orn-Orn, Gln-Arg-Gly-Arg-Ala-Glu-Glu-Val-Tyr-Tyr-Ser-Gly-Thr, and Glu-(γ-spermide)-Arg-Gly-Arg-Ala-Glu-Glu-Val-Tyr-Tyr-Ser-Gly-Thr; and Glu(Arg-Gly-Arg-Ala-Glu-Glu-Val-Tyr-Tyr-Ser-Gly-Thr)-γ-spermide;
u is CH₂ or S;
v is NH o S;
w is CH₂ or CO;
with the proviso that when R₉ is CONH₂, then,
(a) R₅ or R₆ is -CH(CH₃)(OH), or
(b) R₅ and R₆ are H; or
(c) the configuration of the C atom bound to R₉ is S or
(d) the configuration of the C atom bound to R₅ is R;
and with the proviso that when R₉ is -CH(CH₃)OH, then R₈ is GF(CH₂)ₙ where n is 3 and GF is -NH-C(=NH)-NH₂, and R₇ is GF(CH₂)ₙ where n is 2 and GF is NH₂.

2. Compound according to claim 1, where u is CH₂, v is NH, Z is CO, R₉ is -CH(CH₃)(OH) and the configuration of the C atom bound to R₉ is S and has the formula (Ia).

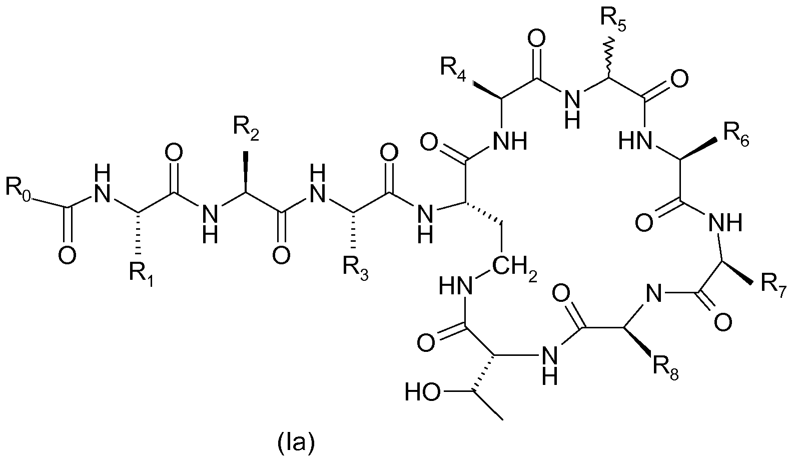

3. Compound according to claim 2 which is nonanoyl-Arg-Thr-Dab-cyclo(4-10)[Dab-Dab-DPhe-Leu-Arg-Dab-Thr].

4. Compound according to claim 2 where u is S, v is S and w is CH₂, and has the formula (Ib)

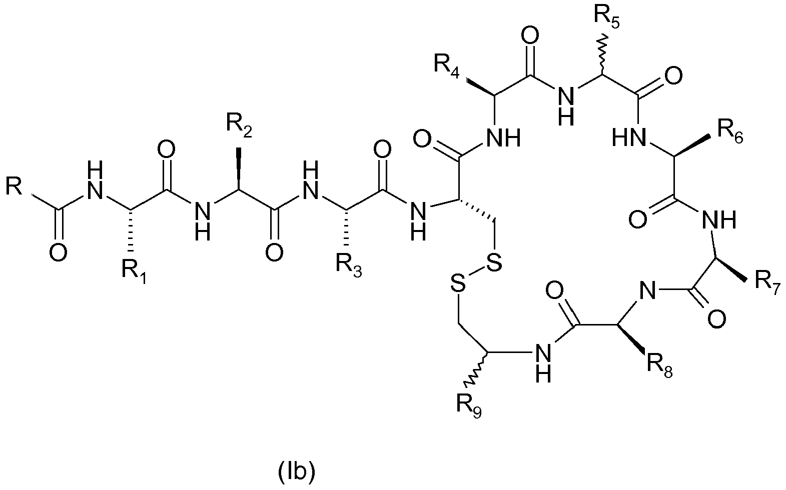

5. Compound according to claim 4, where R₁₁ is a peptide sequence selected from the group that consists of Ala-Leu-Arg, Ala-Leu-Arg-Ala-Leu-Arg, Gly-Arg-Val-Glu-Val-Leu-Tyr-Arg-Gly- Ser-Trp, Lys-Val-Leu, Lys-Val-Leu-Lys-Val-Leu, Leu-Met-Trp-Trp-Met-Leu, Orn-Orn-Orn, Gln-Arg-Gly-Arg-Ala-Glu-Glu-Val-Tyr-Tyr-Ser-Gly-Thr, Glu(y-spermide)-Arg-Gly-Arg-Ala-Glu-Glu-Val-Tyr-Tyr-Ser-Gly-Thr, and Glu(Arg-Gly-Arg-Ala-Glu-Glu-Val-Tyr-Tyr-Ser-Gly-Thr)-γ-spermide;

6. Compound according to claim 5, that is selected from the following ones:
nonanoyl-Arg-Thr-Dab-cyclo(S-S)[Cys-Dab-DPhe-Leu-Arg-Dab-Cys]-Ala-Leu-Arg;
nonanoyl-Arg-Thr-Dab-cyclo(S-S)[Cys-Dab-DPhe-Leu-Arg-Dab-Cys]-Ala-Leu-Arg-Ala-Leu-Arg;
nonanoyl-Arg-Thr-Dab-cyclo(S-S)[Cys-Dab-DPhe-Leu-Arg-Dab-Cys]-Gly-Arg-Val-Glu-Val-Leu-Tyr-Arg-Gly-Ser-Trp];
nonanoyl-Arg-Thr-Dab-cyclo(S-S)[Cys-Dab-DPhe-Leu-Arg-Dab-Cys]-Lys-Val-Leu;
nonanoyl-Arg-Thr-Dab-cyclo(S-S)[Cys-Dab-Phe-Leu-Arg-Dab-Cys]-Lys-Val-Leu-Lys-Val-Leu;
nonanoyl-Arg-Thr-Dab-cyclo(S-S)[Cys-Dab-DPhe-Leu-Arg-Dab-Cys]-Leu-Met-Trp-Trp-Met-Leu;
nonanoyl-Arg-Thr-Dab-cyclo(S-S)[Cys-Dab-DPhe-Leu-Arg-Dab-Cys]-Orn-Orn-Orn;
nonanoyl-Arg-Thr-Dab-cyclo(S-S)[Cys-Dab-DPhe-Leu-Dab-Arg-Cys]-Gln-Arg-Gly-Arg-Ala-Glu-Glu-Val-Tyr-Tyr-Ser-Gly-Thr; and
nonanoil-Arg-Thr-Dab-ciclo(S-S)[Cys-Dab-DPhe-Leu-Dab-Arg-Cys]-Glu-(γ-spermide)-Arg-Gly-Arg-Ala-Glu-Glu-Val-Tyr-Tyr-Ser-Gly-Thr.

7. Compound according to claim 4, that is selected from the following ones:
nonanoyl-Arg-Thr-Arg-cyclo(S-S)[Cys-Dab-Phe-Leu-Arg-Dab-Cys];
nonanoyl-Arg-Thr-Arg-cyclo(S-S)[Cys-Dab-DPhe-Leu-Arg-Dab-DCys];
nonanoyl-Arg-Thr-Dab-cyclo(S-S)[Cys-Dab-Phe-Leu-Arg-Dab-Cys]; and
nonanoyl-Arg-Thr-Dab-cyclo(S-S)[Cys-Dab-DPhe-Leu-Arg-Dab-DCys].

8. Compound according to claim 7, that is selected from the following ones:
nonanoyl-Arg-Thr-Arg-cyclo(S-S)[Cys-Dab-Phe-Leu-Arg-Dab-Cys]; and
nonanoyl-Arg-Thr-Dab-cyclo(S-S)[Cys-Dab-Phe-Leu-Arg-Dab-Cys].

9. Compound according to claim 4, that is selected from the following ones:
nonanoyl-Arg-Thr-Arg-cyclo(S-S)[Cys-Dab-DPhe-Thr-Arg-Dab-Cys];
nonanoyl-Arg-Thr-Arg-cyclo(S-S)[Cys-Dab-Phe-Thr-Arg-Dab-Cys];
nonanoyl-Arg-Thr-Dab-cyclo(S-S)[Cys-Dab-DPhe-Thr-Arg-Dab-Cys];
nonanoyl-Arg-Thr-Dab-cyclo(S-S)[Cys-Dab-Phe-Thr-Arg-Dab-Cys];
nonanoyl-Arg-Thr-Arg-cyclo(S-S)[Cys-Dab-Trp-Thr-Arg-Dab-Cys];
nonanoyl-Arg-Thr-Dab-cyclo(S-S)[Cys-Dab-Trp-Thr-Arg-Dab-Cys];
nonanoyl-Arg-Thr-Arg-cyclo(S-S)[Cys-Dab-DLeu-Thr-Arg-Dab-Cys];
nonanoyl-Arg-Thr-Dab-cyclo(S-S)[Cys-Dab-DLeu-Thr-Arg-Dab-Cys];
decanoyl-Arg-Thr-Arg-cyclo(S-S)[Cys-Dab-DTrp-Thr-Dab-Dab-Cys];
decanoyl-Arg-Thr-Dab-cyclo(S-S)[Cys-Dab-DLeu-Thr-Arg-Dab-Cys];
decanoyl-Arg-Thr-Dab-cyclo(S-S)[Cys-Dab-Trp-Thr-Arg-Dab-Cys]; and
dodecanoyl-Arg-Thr-Dab-cyclo(S-S)[Cys-Dab-Phe-Thr-Arg-Dab-Cys].

10. Compound according to claim 1, that is selected from the following ones:
nonanoyl-Arg-Thr-Dab-cyclo(4-10)[Dab-Dab-DPhe-Leu-Arg-Dab-Thr];
nonanoyl-Arg-Thr-Dab-cyclo(S-S)[Cys-Dab-DPhe-Leu-Arg-Dab-Cys]-Gly-Arg-Val-Glu-Val-Leu-Tyr-Arg-Gly-Ser-Trp];
nonanoyl-Arg-Thr-Dab-cyclo(S-S)[Cys-Dab-DPhe-Leu-Arg-Dab-Cys]-Leu-Met-Trp-Trp-Met-Leu; and
nonanoyl-Arg-Thr-Dab-cyclo(S-S)[Cys-Dab-DPhe-Leu-Arg-Dab-DCyS].

11. Use of a compound as defined in any of the claims 1-10, for the preparation of a medicament for the treatment of a bacterial infection caused by a Gram positive bacteria in a mammal including a human.

12. Use according to claim 11, where the Gram positive bacteria are selected from the group consisting of *Micobacterium phlei, Staphylococcus aureus,* and *Micrococcus luteus.*

13. Use of a compound as defined in any of the claims 1-10, for the preparation of a medicament for the treatment of a bacterial infection caused by a Gram negative bacteria in a mammal including a human.

14. Use according to claim 13, where the Gram negative bacteria are selected from the group consisting of Salmonella typhimurium, Pseudomonas aeruginosa, Escherichia coli and Acinetobacter sp.

15. A pharmaceutical composition that comprises a therapeutically efective amount of a compound as defined in any of the claims 1-10 together with pharmaceutically acceptable excipients or carriers.
